# EUROPÄISCHE PATENTANMELDUNG

(11) **EP 2 869 258 A1**
(43) Veröffentlichungstag der Anmeldung: **06.05.2015**
(21) Anmeldenummer: 13005160.0
(22) Anmeldetag: 31.10.2013
(51) Int. Cl.: G06Q 50/24, G06Q 10/10

(54) **Verfahren zur Übermittlung eines Auftrags mit Laboranforderungen**

(71) Anmelder: Imedac GmbH, 32105 Bad Salzuflen (DE)
(72) Erfinder: Bäumer, Sascha, 32657 Lemgo (DE); Münstermann, Dieter, 32049 Herford (DE)
(74) Vertreter: Kayser, Christoph

(57) **Zusammenfassung**

Ein Verfahren zur Übermittlung eines Auftrags mit Laboranforderungen umfasst die Schritte
- Bereitstellen eines ersten digitalen Datensatzes beim Auftraggeber, der einem ersten Formular mit Laboranforderungen entspricht;
Anzeigen des ersten Formulars auf einem Bildschirm des Auftraggebers in einem manipulierbaren Format;
- Manipulieren des ersten Formulars durch optisches Markieren wenigstens einer Laboranforderung des ersten Formulars;
- Erzeugen eines zweiten digitalen Datensatzes beim Auftraggeber, der dem optisch markierten ersten Formular entspricht;
- Erzeugen eines Überweisungsscheins an den Auftragnehmer auf der Basis des zweiten digitalen Datensatzes, auf dem auch patientenbezogene Daten enthalten sind; und
- Übermitteln des Überweisungsscheins an den Auftragnehmer.

## Beschreibung

Die vorliegende Erfindung betrifft ein Verfahren zur Übermittlung eines Auftrags mit Laboranforderungen von einem Auftraggeber an einen Auftragnehmer.

Ein solches Verfahren ist aus dem Stand der Technik allgemein bekannt. Ein übliches Verfahren des Standes der Technik besteht darin, z. B. einem Laboratorium einen solchen Auftrag mithilfe eines vorgedruckten Formulars zu erteilen, auf dem mithilfe einer Strichmarkierung, zum Beispiel einem sogenannten OMR Code (Optical Mark Recognition Code) personenbezogene Patientendaten zur Patientenidentifikation aufgebracht sind.

Die zu einem solchen Auftrag gehörenden Proben werden mithilfe von Barcodes identifiziert, die auf dem jeweiligen Probengefäß angebracht sind. Die ersten Stellen eines solchen Barcodes, zum Beispiel ein zehnstelliger Teil dieses Barcodes, bilden die eindeutige Auftragsidentifizierung und werden durch einen Teil des Barcodes ergänzt, der das Probenmaterial und somit die einzelnen Probengefäße dieses Auftrags identifiziert. Die Barcodes werden mehrfach erzeugt, so dass einer dieser Barcodes auf dem Auftragsformular durch Aufkleben dauerhaft fixiert und ein anderer am Probengefäß angebracht werden kann. Damit wird ein Zusammenhang zwischen Probengefäß bzw. Probe und Auftrag hergestellt.

Das Auftragsformular wird beim Auftragnehmer, z.B. einem ausführenden Labor, maschinell gelesen und in eine Datenverarbeitungsanlage des ausführenden Labors übertragen.

Obwohl mit einem solchen Verfahren des Standes der Technik bereits weitgehend maschinell gearbeitet wird, können durch fehlerhaft erstellte und/oder fehlerhaft gelesene Formulare Informationen falsch übertragen und/oder Zuordnungen zwischen Auftrag und Probe unrichtig hergestellt werden. Zur Vermeidung solcher Fehler bleibt in jedem Falle die Notwendigkeit einer visuellen Kontrolle und gegebenenfalls einer manuellen Nachbesserung bestehen.

Ein Nachteil der Verfahren des Standes der Technik besteht somit darin, dass sie sehr aufwändig sind, sowohl hinsichtlich der Zeit als auch hinsichtlich des Personalbedarfs für die Kontrolle, so dass die Erfassung eines Auftrags relativ lange dauert und nicht völlig fehlerfrei durchzuführen ist.

Ein weiterer Nachteil besteht auch darin, dass durch die nach wie vor aufwändige Bearbeitung Laboraufträge nur verzögert ausgeführt werden können und somit ein daraus erzeugter Befundbericht für den Auftraggeber nur mit einiger Verspätung zur Verfügung gestellt werden kann.

Die Aufgabe der Erfindung ist daher, das Verfahren der eingangs genannten Art derart weiterzubilden, dass dieses insgesamt weniger Zeit benötigt und fehlerfrei durchgeführt werden kann.

Die Aufgabe wird erfindungsgemäß durch die Merkmale des Anspruchs 1 gelöst.

So ist es von Vorteil, dass mit der vorliegenden Erfindung ein erster digitaler Datensatz bereitgestellt wird, der einem Formular mit Laboranforderungen entspricht und dieses Formular auf einem Bildschirm angezeigt wird, wobei das Formular in einem manipulierbaren Format angezeigt wird und der Auftraggeber das Formular durch optisches Markieren wenigstens einer Laboranforderung manipulieren kann. Dadurch wird ein zweiter digitaler Datensatz beim Auftraggeber erzeugt, der dem optisch markierten Formular entspricht, wobei dieses dann als Überweisungsschein an den Auftragnehmer auf der Basis des zweiten digitalen Datensatzes zusammen mit patientenbezogenen Daten erzeugt und an den Auftragnehmer übermittelt wird. Die Übermittlung kann in beliebiger Weise erfolgen, z. B. per E-Mail, Telefax, Boten und/oder per Postversand.

Ein weiterer Vorteil der vorliegenden Erfindung ist, dass der erzeugte Überweisungsschein beim Auftraggeber ausgedruckt und an den Auftragnehmer übersandt werden kann.

Ein weiterer Vorteil der vorliegenden Erfindung ist, dass der Schritt des Erzeugens eines zweiten digitalen Datensatzes zusätzlich ein Umwandeln wenigstens eines Teils des optisch markierten ersten Formulars in einen 2D Barcode mit PDF417 Symbologie umfasst.

Dadurch ist gewährleistet, dass die erzeugten patientenbezogenen Daten nicht nur in Klarschrift, sondern auch verschlüsselt vorliegen.

Ein weiterer Vorteil der vorliegenden Erfindung ist, dass der Schritt des Erzeugens eines zweiten digitalen Datensatzes zusätzlich ein Umwandeln wenigstens eines Teils des optisch markierten ersten Formulars in einen zweiten 2D Barcode mit PDF417 Symbologie umfasst.

Ein solcher zweiter 2D Barcode enthält die Auftragsdaten und ist für jeden Auftragnehmer spezifisch. Durch die Verwendung von zwei 2D Barcodes können Fehllesungen ausgeschlossen werden.

Ein weiterer Vorteil der vorliegenden Erfindung ist, dass die patientenbezogenen Daten, wie Name, Geburtstag, Diagnose usw. per Knopfdruck aus der Datenverarbeitungsanlage des Auftraggebers auf den Überweisungsschein übernommen werden können. Dies vermeidet Übertragungsfehler und beschleunigt den Vorgang.

Des Weiteren können diese Daten auch manuell in das erste Formular eingetragen werden.

Ein weiterer Vorteil der vorliegenden Erfindung ist, dass die Anforderungen in die Datenverarbeitungsanlage des Auftraggebers automatisch übernommen und der Patientenakte hinzugefügt werden. Es besteht somit Datenintegrität. Es erfolgt also eine Datenübernahme der Patientendaten aus dem IT-System des Auftraggebers und ein Zurückschreiben der Auftragsdaten in die "elektronische Patientenakte" im IT-System des Auftraggebers.

Die vorliegende Erfindung ermöglicht zudem eine Arbeit mit nahezu allen existierenden Datenverarbeitungsanlagen bzw. IT-Systemen der jeweiligen Auftraggeber.

Zudem wird in vorteilhafter Weise eine Umstellung auf eine völlig papierlose Übermittlung mit minimaler Anpassung möglich. Daher umfasst die vorliegende Erfindung auch, dass auf den Ausdruck verzichtet und der Auftrag auf elektronischem Wege übertragen wird.

Weitere Vorteile der vorliegenden Erfindung ergeben sich aus den Merkmalen der weiteren Unteransprüche.

Eine Ausführungsform der vorliegenden Erfindung wird im Folgenden anhand der Zeichnungen näher beschrieben. Es zeigen:
Fig. 1 eine schematische Darstellung eines Auftragsformulars auf dem Bildschirm eines Datenverarbeitungsgerätes des Auftraggebers; und
Fig. 2 eine schematische Darstellung des ausgedruckten Überweisungsscheins an den Auftragnehmer.

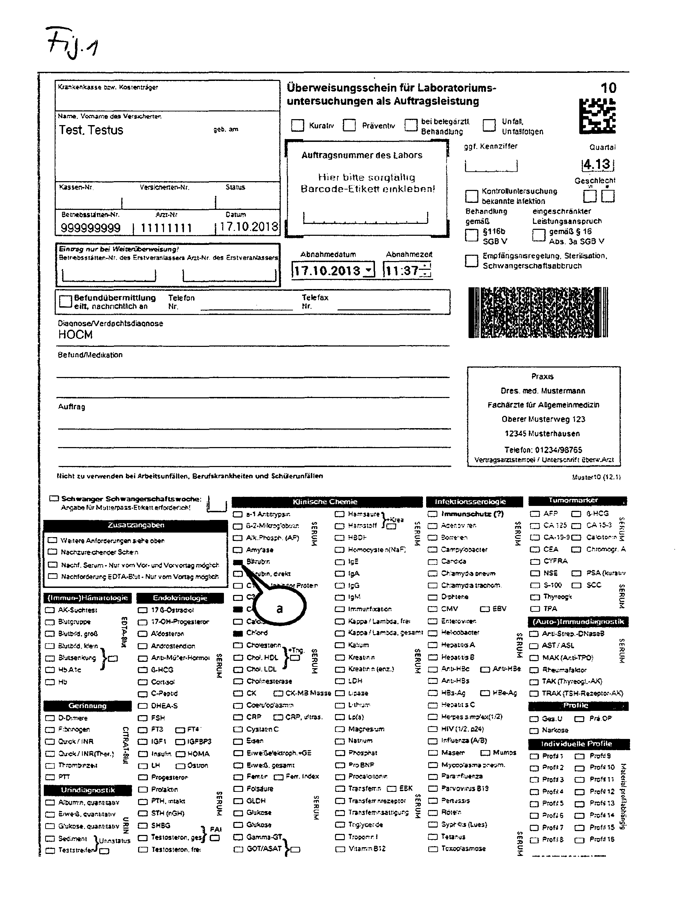

Das erfindungsgemäße Verfahren wird mithilfe von im Handel erhältlichen Datenverarbeitungseinrichtungen durchgeführt. Zur Durchführung des erfindungsgemäßen Verfahrens steht bei einem Auftraggeber eine Datenverarbeitungseinrichtung, auf deren Bildschirm anhand eines ersten digitalen Datensatzes ein Formular mit Laboranforderungen angezeigt werden kann. Dieses Formular wird auf dem Bildschirm des Auftraggebers in Abhängigkeit von einem ersten digitalen Datensatz in einem Format angezeigt, das manipulierbar bzw. editierbar ist. Der Auftraggeber kann dann auf dem am Bildschirm angezeigten Formular Laboranforderungen optisch markieren. Das ist in Fig. 1 durch die geschwärzten Felder und das Bezugszeichen a dargestellt.

Auf diese Weise wird ein zweiter digitaler Datensatz erzeugt, der dem optisch markierten Formular, also von einem Bediener manipulierten Formular, entspricht. Im oberen Teil dieses Formulars in Fig.1 sind patientenbezogene Daten in Klarschrift enthalten.

Im Folgenden ist mit dem Begriff "erster digitaler Datensatz" ein digitaler Datensatz gemeint, mit dem das erste Formular aus Fig. 1 ohne eine optische Markierung dargestellt wird. Mit dem Begriff "zweiter digitaler Datensatz" ist im Folgenden ein Datensatz gemeint, mit dem das erste Formular aus Fig. 1 mit einer vom Auftraggeber durchgeführten optischen Markierung und ggf. mit wenigstens einem 2D Barcode dargestellt ist.

Da in Fig. 1 die optische Markierung bereits vorgenommen wurde, zeigt Fig. 1 somit ein optisch markiertes erstes Formular auf der Basis des zweiten digitalen Datensatzes.

Zur Erzeugung eines solchen editierbaren Formulars ist auf der Datenverarbeitungseinrichtung eine gängige Software installiert, wie zum Beispiel ein Softwareprodukt der Adobe Acrobat-Familie von Adobe Systems Corporation, San Jose, Kalifornien, USA.

Mit dem Erzeugen des zweiten digitalen Datensatzes, das heißt, infolge der optischen Markierung von wenigstens einer Laboranforderung, wird wenigstens ein Teil des optisch markierten ersten Formulars in einen ersten 2D Barcode mit PDF417 Symbologie umgewandelt. Dieser 2D Barcode enthält patientenbezogene Daten und dazugehörige Auftragsdaten.

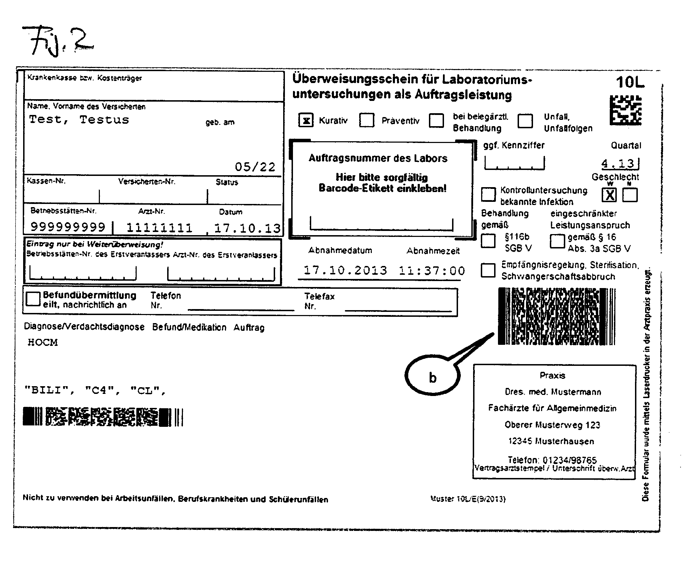

In einer bevorzugten Ausführungsform wird zusätzlich ein zweiter 2D Barcode mit PDF417 Symbologie erzeugt, wie dies in Fig. 2 dargestellt ist. Dieser zweite 2D Barcode umfasst die Auftragsdaten.

Das optisch markierte Formular aus Fig. 1 und der Überweisungsschein aus Fig. 2 können dann in unterschiedlicher Weise an den Auftragnehmer übermittelt werden.

In einer bevorzugten Ausführungsform wird der Überweisungsschein aus Fig. 2 ausgedruckt und an den Auftragnehmer übersandt. Dazu wird dem Auftraggeber ein zertifiziertes Blankoformular zur Verfügung gestellt, auf das der Überweisungsschein gedruckt wird.

Ein Zusammenhang zu Probengefäßen wird wie in den bisherigen Verfahren des Standes der Technik hergestellt. Dies erfolgt regelmäßig durch Barcodes, die auf das optisch markierte Formular geklebt werden und auf ein entsprechendes Probengefäß geklebt werden.

Die Barcodeetiketten sind auf dem optisch markierten Formular sowie auf dem entsprechenden Probengefäß dauerhaft fest angebracht.

Für die Zuordnung erhält der Auftraggeber entweder die Barcodeetiketten in geeigneter Form und eindeutiger Nummerierung vom Auftragnehmer zur freien Verfügung im erfindungsgemäßen Verfahren oder der Auftraggeber druckt die Barcodeetiketten vor Ort selbst. In diesem Falle wird durch eine entsprechende Information aus dem Datenverarbeitungssystem des Auftragnehmers die Eindeutigkeit der Proben- und Auftragsidentifikationsnummer sichergestellt.

## Patentansprüche

1. Verfahren zur Übermittlung eines Auftrags mit Laboranforderungen, mit den Schritten:
- Bereitstellen einer Datenverarbeitungseinrichtung aus einer Mehrzahl von IT-Systemen;
- Bereitstellen eines ersten digitalen Datensatzes beim Auftraggeber, der einem ersten Formular mit Laboranforderungen entspricht und in der Datenverarbeitungseinrichtung bearbeitet werden kann;
Anzeigen des Formulars auf einem Bildschirm der Datenverarbeitungseinrichtung des Auftraggebers in einem manipulierbaren Format;
- Manipulieren des Formulars durch optisches Markieren wenigstens einer Laboranforderung des Formulars;
- Erzeugen eines zweiten digitalen Datensatzes beim Auftraggeber, der dem optisch markierten Formular entspricht;
- Erzeugen eines Überweisungsscheins an den Auftragnehmer auf der Basis des zweiten digitalen Datensatzes, auf dem auch patientenbezogene Daten enthalten sind; und
- Übermitteln des Überweisungsscheins an den Auftragnehmer.

2. Verfahren nach Anspruch 1,
**dadurch gekennzeichnet,**
**dass** der Schritt des Übermittelns auch ein Drucken des Überweisungsscheins beim Auftraggeber und ein Übersenden des gedruckten Überweisungsscheins an den Auftragnehmer umfasst.

3. Verfahren nach Anspruch 1 oder 2,
**dadurch gekennzeichnet,**
**dass** der Schritt des Erzeugens eines zweiten digitalen Datensatzes zusätzlich ein Umwandeln des optisch markierten Formulars in einen ersten 2D-Barcode mit PDF417 Symbologie umfasst.

4. Verfahren nach einem der Ansprüche 1 bis 3,
**dadurch gekennzeichnet,**
**dass** der Schritt des Erzeugens eines zweiten digitalen Datensatzes zusätzlich ein Umwandeln des ersten digitalen Datensatzes in einen zweiten 2D-Barcode mit PDF417 Symbologie umfasst.

5. Verfahren nach einem der Ansprüche 2 bis 4,
**dadurch gekennzeichnet,**
**dass** vor dem Drucken ein zertifiziertes Blankoformular bereitgestellt wird, auf den der Überweisungsschein gedruckt wird.

6. Verfahren nach einem der Ansprüche 1 bis 5,
**dadurch gekennzeichnet,**
**dass** die patientenbezogenen Daten in Klarschrift erzeugt werden.

7. Verfahren nach einem der Ansprüche 1 bis 6,
**dadurch gekennzeichnet,**
**dass** ein zweiter 2D-Barcode mit den Auftragsdaten gedruckt wird.

8. Verfahren nach einem der vorstehenden Ansprüche,
**dadurch gekennzeichnet,**
**dass** dem Auftraggeber Barcodeetiketten mit eindeutiger Nummerierung des Auftragnehmers bereitgestellt werden.
